(19) Europäisches Patentamt
European Patent Office
Office européen des brevets

(11) Veröffentlichungsnummer : **0 121 054**
**B1**

(12) # EUROPÄISCHE PATENTSCHRIFT

(45) Veröffentlichungstag der Patentschrift : **03.12.86**

(51) Int. Cl.⁴ : **C 07 D233/80**, C 07 D235/02, A 01 N 43/50

(21) Anmeldenummer : **84101202.4**

(22) Anmeldetag : **07.02.84**

(54) **N-sulfenylierte Hydantoine, ein Verfahren zu ihrer Herstellung und ihre Verwendung als Mikrobizide.**

(30) Priorität : **16.02.83 DE 3305203**

(43) Veröffentlichungstag der Anmeldung : **10.10.84 Patentblatt 84/41**

(45) Bekanntmachung des Hinweises auf die Patenterteilung : **03.12.86 Patentblatt 86/49**

(84) Benannte Vertragsstaaten : **BE CH DE FR GB IT LI SE**

(56) Entgegenhaltungen :
**DE-A- 1 168 914**
**DE-A- 2 441 601**
**DE-A- 3 222 523**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Kühle, Engelbert, Dr.**
**von-Bodelschwingh-Strasse 42**
**D-5060 Bergisch Gladbach 2 (DE)**
Erfinder : **Paulus, Wilfried, Dr.**
**Deswatinesstrasse 90**
**D-4150 Krefeld 1 (DE)**
Erfinder : **Genth, Hermann, Dr.**
**Am Heckerhof 60**
**D-4150 Krefeld 1 (DE)**
Erfinder : **Reinecke, Paul, Dr.**
**Lessingstrasse 11**
**D-5090 Leverkussen 3 (DE)**
Erfinder : **Rosslenbroich, Hans-Jürgen, Dr.**
**Rembrandtstrasse 20**
**D-4019 Monheim (DE)**

**EP 0 121 054 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-sulfenylierte Hydantoine, ein Verfahren zu ihrer Herstellung sowie ihre Verwendung in mikrobiziden Mitteln.

Die Verwendung einiger N-(Trihalogenmethylthio)-Verbindungen zum Schutz technischer Materialien gegen mikrobiellen Abbau ist bekannt (US 2 553 770 ; Journ. Agr. Food Chem. *14*, 365 (1966) ; Fette, Seifen, Anstrichmittel *68*, 272 (1966)). Bei ihrer Anwendung treten jedoch bisweilen Probleme auf, da die bekannten Mittel sich in Anstrich- und Imprägniermitteln schlecht lösen.

Aus der DE-AS 11 68 914 ist ein Verfahren zur Herstellung von 3-N-Fluordichlormethylthiohydantoinen bekannt, die fungizide Eigenschaften besitzen und daher als Schädlingsbekämpfungs- bzw. Pflanzenschutzmittel Verwendung finden können. Weiterhin sind aus der DE-OS 24 41 601 neue Imidazolidin-2,4-dion-Derivate bekannt, die zur Bekämpfung von Schädlingen eingesetzt werden können.

Es wurden neue N-sulfenylierte Hydantoine der Formel

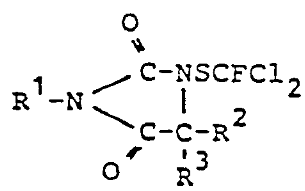

(I)

gefunden, in der

$R^1$ einen Alkylrest mit 1-12 C-Atomen, einen Alkenylrest mit 3-8 C-Atomen, einen Cycloalkylrest mit 5-12 C-Atomen oder einen Aralkylrest mit 6-10 C-Atomen im aromatischen Teil und 1-6 C-Atomen im aliphatischen Teil bedeutet, die gegebenenfalls durch 1-3 gleiche oder verschiedene Substituenten aus der Gruppe Alkylreste mit 1-4 C-Atomen und/oder Fluor-, Chlor-, Brom- oder Iodatomen substituiert sein können, wobei die Alkylsubstituenten noch durch Sauerstoff- oder Schwefelatomen unterbrochen sein können, oder

$R^1$ für

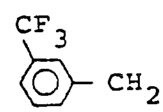

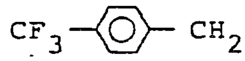

$C_6H_{11}CH_2$

oder

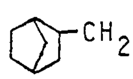

steht und

$R^2$ und $R^3$ gleich oder verschieden sind und für Wasserstoff, einen Alkylrest mit 1-12 C-Atomen oder einen Alkenylrest mit 3-8 C-Atomen stehen oder zu einem Cycloalkylrest mit 5-12 C-Atomen verbunden sind.

Die neuen N-sulfenylierten Hydantoine haben neben einer hervorragenden mikrobiziden Wirkung eine gute Löslichkeit in organischen Lösungsmitteln und eignen sich besonders zum Schutz technischer Materialien vor mikrobieller Zersetzung oder Zerstörung und im Pflanzenschutz.

Alkyl bedeutet erfindungsgemäß einen geradkettigen oder verzweigten Kohlenwasserstoffrest mit 1-8 Kohlenstoffatomen. Beispielsweise einen Methyl-, Ethyl-, Isopropyl-, Butyl-, Neopentyl-, Hexyl- und Octyl-Rest.

Alkenyl bedeutet erfindungsgemäß einen geradkettigen oder verzweigten ungesättigten Kohlenwasserstoffrest mit 3-8 Kohlenstoffatomen und einer oder zwei, bevorzugt einer, Doppelbindung. Beispielsweise seien Allyl, Crotonyl und Isooctenyl genannt.

Cycloalkyl bedeutet erfindungsgemäß einen cyclischen Kohlenwasserstoffrest mit 5-8 Kohlenstoffatomen. Beispielsweise seien genannt : Cyclopentyl, Cyclohexyl, Cycloheptyl und Cyclooctyl.

Aralkyl bedeutet einen aus einem aromatischen und einem aliphatischen Teil bestehenden Rest, wobei der aromatische Teil aus 6 bis 10 Kohlenstoffatomen, bevorzugt aus Phenyl und der aliphatische Teil aus 1 bis 6 Kohlenstoffatomen, besteht. Beispielsweise seien die folgenden Aralkylreste genannt : Benzyl, 2-Methylbenzyl, 2-Phenethyl und 1-Phenethyl.

Die Alkyl-, Alkenyl-, Cycloalkyl- und Aralkylreste können insbesondere 1 oder 2 gleiche oder verschiedene Substituenten tragen. Als Substituenten seien aufgeführt : Alkyl mit 1 bis 4, insbesondere 1 oder 2 Kohlenstoffatomen, wie Methyl, Ethyl, n- und iso-Propyl ; Fluor, Chlor, Brom und Iod, insbesondere Chlor und Brom ; die Alkylreste können außerdem durch Sauerstoff oder Schwefel unterbrochen sein.

Im einzelnen seien die folgenden sulfenylierten Hydantoine genannt: Die 1-(Dichlorfluormethylmercapto)-derivate von 3-Methyl, 3-Ethyl, 3-Isopropyl, 3-Methoxyethyl-, 3-Ethylmercaptoethyl-, 3-tert.-Butyl-, 3-Neopentyl-, 3-Cyclopentyl-, 3-(4-Methylcyclohexyl)-, 3-(4-Chlorbenzyl)-, 3(3-Chlorphenethyl)-, 3,5,5-Trimethyl-, 3-Cyclohexyl, 5,5-Dimethyl-, 3-Cyclohexyl-diethyl- und 3-Cyclohexyl-5-butylhydantoin.

Es wurde auch ein Verfahren zur Herstellung von N-sulfenylierten Hydantoinen der Formel (I) gefunden, daß dadurch gekennzeichnet ist, daß man Hydantoine der Formel

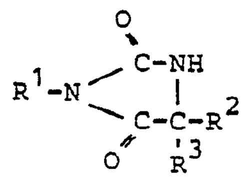

(II)

in der $R^1$, $R^2$ und $R^3$ die oben genannte Bedeutung haben, mit Dichlorfluormethansulfenylchlorid der Formel

$$FCl_2CSCl \quad (III),$$

in Gegenwart eines Verdünnungsmittels und eines säurebindenden Mittels umsetzt.

Das erfindungsgemäße Verfahren kann durch das folgende Formelbild erläutert werden :

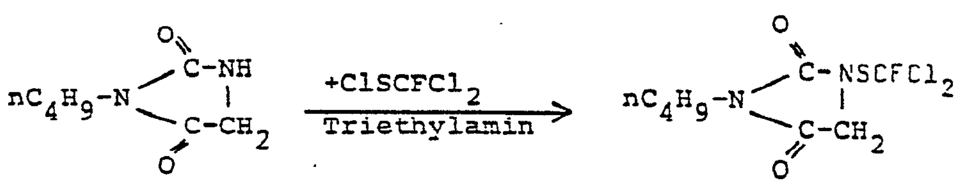


Die für das erfindungsgemäße Verfahren zu verwendenden Hydantoine sind an sich bekannt und lassen sich in an sich bekannter Weise herstellen, z. B. indem man

a) Isocyanate mit Aminoessigsäure umsetzt und anschließend das Additionsprodukt durch Dehydratisierung cyclisiert, oder

b) Isocyanate mit α-Aminonitril umsetzt und anschließend das Additionsprodukt durch Erhitzen mit konzentrierter Salzsäure cyclisiert und hydrolysiert (CA *55*, 27277 a (1961)).

Dichlorfluormethansulfenylchlorid ist ebenfalls bekannt (Ang. Chem. *76*, 807 (1964)). Bei der Durchführung des erfindungsgemäßen Verfahrens kommen als Verdünnungsmittel alle inerten organischen Lösungsmittel in Frage. Hierzu gehören vorzugsweise Kohlenwasserstoffe wie Toluol, Chlorkohlenwasserstoffe wie Chlorbenzol, Ether wie Dioxan. Man kann die Umsetzung aber auch in Wasser durchführen.

Als säurebindende Mittel können tertiäre Amine sowie Alkalihydroxide oder Alkalicarbonate verwendet werden.

Tertiäre Amine sind beispielsweise Verbindungen der Formel

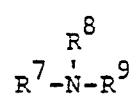

(IV)

in der $R^7$, $R^8$ und $R^9$ gleich oder verschieden sind und für Niederalkyl ($C_1$ bis etwa $C_6$) stehen.

Beispielsweise seien die folgenden tertiären Amine genannt : Trimethyl-, Triethyl- und Dimethylbenzylamin.

Alkalihydroxide und Alkalicarbonate sind im wesentlichen Natrium- und Kaliumhydroxid und Natrium- und Kaliumcarbonat.

Das erfindungsgemäße Verfahren wird im allgemeinen im Temperaturbereich von 0 bis 100 °C, bevorzugt 20 bis 50 °C, durchgeführt.

Das erfindungsgemäße Verfahren wird im allgemeinen bei Normaldruck durchgeführt. Es ist aber auch möglich, das Verfahren bei einem Über- oder Unterdruck durchzuführen.

Bei der Durchführung des erfindungsgemäßen Verfahrens setzt man im allgemeinen auf 1 Mol des entsprechenden Hydantoins 1 bis 3, bevorzugt 1 bis 1,1 Mol des Dichlorfluormethansulfenylchlorids ein.

Das erfindungsgemäße Verfahren kann im allgemeinen wie folgt durchgeführt werden :

Das Hydantoin wird unter Zugabe des Säurebindemittels in dem Verdünnungsmittel gelöst und mit Dichlor-fluor-methansulfenylchlorid versetzt. Zur Umsetzung hält man das Reaktionsgemisch in dem erfindungsgemäßen Temperaturbereich. Nach Abtrennung des säurebindenden Mittels engt man die Lösung ein und trennt das Reaktionsprodukt ab.

Die erfindungsgemäßen N-sulfenylierten Hydantoine sind Wirkstoffe zur Bekämpfung von Mikroorganismen im Materialschutz und Pflanzenschutz. Unter Materialschutz versteht man erfindungsgemäß den Schutz technischer Materialien vor Veränderung oder Zerstörung durch Mikroorganismen.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die durch Mikroorganismen zersetzt werden können.

Technische Materialien, die durch die erfindungsgemäßen Wirkstoffe vor einer mikrobiellen Veränderung oder Zerstörung geschützt werden sollen, sind beispielsweise Klebstoffe, Leime, Papiere und Kartone, Textilien, Leder, Holz, Anstrichmittel und Kunststoffartikel, die von Mikroorganismen befallen und zersetzt werden können. Im Rahmen der zu schützenden Materialien seien auch Teile von Produktionsanlagen, wie beispielsweise Kühlwasser- und Kühlschmiermittelkreisläufe genannt, deren Funktionstüchtigkeit durch Mikroorganismen beeinträchtigt werden kann. Vorzugsweise können die erfindungsgemäßen Wirkstoffe zum Schutz von Holz oder Anstrichmitteln verwendet werden.

Mikroorganismen, die einen Abbau oder eine Veränderung der technischen Materialien bewirken können, sind beispielsweise Bakterien, Pilze, Hefen, Algen und Schleimorganismen. Vorzugsweise wirken die erfindungsgemäßen Wirkstoffe gegen Schimmelpilze, holzverfärbende Pilze und holzzerstörende Pilze (Basidiomyceten) sowie gegen Schleimorganismen.

Es seien beispielsweise Mikroorganismen der folgenden Gattungen genannt :

Alternaria, wie Alternaria tenuis,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Staphylococcus, wie Staphylococcus aureus.

Je nach ihrem Anwendungsgebiet können die erfindungsgemäßen Wirkstoffe in die üblichen Formulierungen überführt werden, wie Lösungen, Emulsionen, Suspensionen, Pulver, Pasten und Granulate.

Diese können in an sich bekannter Weise hergestellt werden, z. B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Verstreckmitteln, gegebenenfalls organische Lösungsmittel, als Hilfslösungsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichlorethan, sein. Die Anwendungskonzentration der erfindungsgemäßen Wirkstoffe richtet sich nach der Art und dem Vorkommen der zu bekämpfenden Mikroorganismen sowie nach der Zusammensetzung des zu schützenden Materials. Die optimale Einsatzmenge kann durch Testreihen leicht ermittelt werden. Im allgemeinen liegen die Anwendungskonzentrationen im Bereich von 0,001 bis 5 Gew.-%, vorzugsweise von 0,05 bis 2,0 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt : Benzimidazolylmethylcarbamate, Tetramethyl-thiuramdisulfid, Zinksalze von Dialkyldithiocarbamaten, 2,4,5,6-Tetrachlor-isophthalonitril, Thiazolylbenzimidazol, Mercaptobenzthiazol und Phenolderivate, wie 2-Phenylphenol, (2,2'-Dihydroxy-5,5'-dichlor)-diphenylmethan.

Die erfindungsgemäßen Wirkstoffe können auch als Schädlingsbekämpfungsmittel im Pflanzenschutz verwendet werden.

Sie können z. B. als fungizide Mittel zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nicht-ionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Herstellungsbeispiele

### Beispiel 1

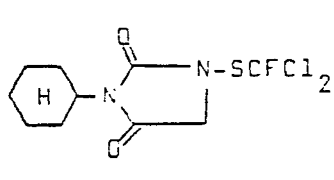

19,4 g (0,106 Mol) 3-Cyclohexyl-hydantoin werden unter Zugabe von 11 g (0,11 Mol) Triethylamin in 120 ml Dioxan gelöst und bei Raumtemperatur tropfenweise mit 17,7 g (0,105 Mol) Dichlor-Fluormethansulfenylchlorid versetzt. Die Temperatur steigt hierbei bis etwa 45 °C an. Man saugt in der Kälte das ausgefallene tert.-Aminhydrochlorid ab, engt das Filtrat im Vakuum ein und kristallisiert das rohe Reaktionsprodukt (33 g) aus Ethanol um. Man erhält 14 g = 42 % der Theorie an 1-(Dichlorfluormethylmercapto)-3-cyclohexyl-hydantoin vom Schmp. 101-103 °C.

In analoger Weise erhält man die folgenden Verbindungen :

(Siehe Tabelle Seite 7 f.)

## Anwendungsbeispiele

### Beispiel 26

Zum Nachweis der Wirksamkeit gegen Pilze werden die minimalen Hemmkonzentrationen (MHK) von erfindungsgemäßen Wirkstoffen bestimmt :

Ein Agar, der aus Bierwürze und Pepton hergestellt wird, wird mit erfindungsgemäßen Wirkstoffen in Konzentrationen von 0,1 mg/l bis 5 000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt ; sie ist in der nachstehenden Tabelle angegeben.

Tabelle I

Angabe der MHK-Werte in mg/l bei der Einwirkung von N-sulfenylierten Hydantoinen auf Pilze

| | Testsubstanz gemäß | | |
|---|---|---|---|
| Testorganismen | Bsp. 3 | Bsp. 5 | Bsp. 10 |
| Alternaria tenuis | 1,5 | 2,0 | 3,5 |
| Aspergillus niger | 10,0 | 50,0 | 20,0 |
| Aureobasidium pullulans | 1,0 | 2,0 | 3,5 |
| Chaetomium globosum | 1,5 | 3,5 | 1,5 |
| Coniophora cerebella | 1,0 | 0,75 | 0,75 |
| Lentinus tigrinus | 1,0 | 0,75 | 0,75 |
| Penicillium glaucum | 50,0 | 100,0 | 75,0 |
| Polyporus versicolor | 7,5 | 15,0 | 15,0 |
| Sclerophoma pityophila | 5,0 | 5,0 | 5,0 |

### Beispiel 27

Wirkung gegen Bakterien

Ein Agar, der als Nährmedium Bouillon enthält, wird mit den in Tabelle II angegebenen Wirkstoffen in Konzentrationen von 1 bis 5 000 ppm versetzt. Darauf infiziert man das Nährmedium mit Staphylococcus aureus und hält das infizierte Medium 2 Wochen bei 28 °C und 60 bis 70 % rel. Luftfeuchtigkeit. Die MHK ist die niedrigste Konzentration an Wirkstoff, bei der keinerlei Bewuchs durch die verwendete Mikrobenart erfolgt.

Die MHK-Werte sind in Tabelle II wiedergegeben.

Tabelle II

Angabe der MHK-Werte in mg/l bei der Einwirkung von N-sulfenylierten Hydantoinen auf Staphylococcus aureus

| Testsubstanz gemäß Beispiel | MHK (mg/l) |
|---|---|
| 3 | 50 |
| 5 | 100 |
| 10 | 50 |

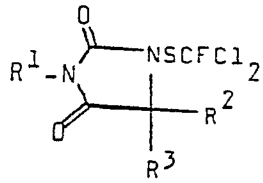

| Beispiel Nr. | $R^1$ | $R^2$ | $R^3$ | Schmp. ($n_D^{20}$) |
|---|---|---|---|---|
| 2 | $CH_3$ | H | H | 88-90°C |
| 3 | $CH_3$ | $CH_3$ | $CH_3$ | ölig |
| 4 | $iC_3H_7$ | H | H | (1,5121) |
| 5 | $nC_4H_9$ | H | H | (1,5182) |
| 6 | $iC_4H_9$ | H | H | 60-64°C |
| 7 | $tC_4H_9$ | H | H | (1,5205) |
| 8 | $(CH_3)_3CCH_2$ | H | H | 98-99°C |
| 9 | $nC_4H_9$ | $CH_3$ | $CH_3$ | ölig |
| 10 | $C_6H_{11}$ | $CH_3$ | $CH_3$ | 73-75°C |
| 11 | $C_6H_5CH_2-$ | H | H | 78° C |
| 12 | $iC_3H_7$ | $CH_3$ | $CH_3$ | 48-49°C |
| 13 | $(CH_3)CCH_2$ | $CH_3$ | $CH_3$ | 59-60°C |
| 14 | $C_6H_5CH_2$ | $CH_3$ | $CH_3$ | 67-69°C |
| 15 | $C_6H_5CH_2CH_2$ | $CH_3$ | $CH_3$ | 86-88°C |
| 16 | $C_6H_5CH_2CH_2$ | $CH_3$ | $CH_3$ | (1.5414) |
| 17 | 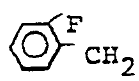 | H | H | 70°C |
| 18 | 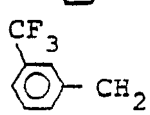 | H | H | 87° C |
| 19 | 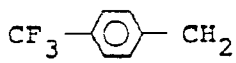 | H | H | 140°C |
| 20 | $C_6H_{11}CH_2$ | $CH_3$ | $CH_3$ | (1.5164) |
| 21 | $C_6H_{11}CH_2$ | H | H | 68-71°C |
| 22 | 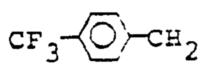 | H | H | 105-107°C |
| 23 | 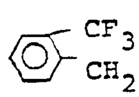 | H | H | 98-99°C |
| 24 | 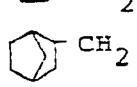 | H | H | (1.5378) |
| 25 | 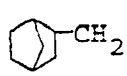 | $CH_3$ | $CH_3$ | 88-91°C |

Beispiel 28 (Wirkung gegen Schleimorganismen)

Die Verbindungen gemäß Beispiel 3, 5 und 10 werden in Konzentrationen von jeweils 0,1 bis 100 mg/l in Allens Nährlösung (Arch. Mikrobiol. *17*, 34-53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

Tabelle III

Angabe der MHK-Werte in mg/l bei der Einwirkung N-sulfenylierter Hydantoine auf Schleimorganismen

| Testsubstanz gemäß Beispiel | MHK in mg/l |
|---|---|
| 3 | 3 |
| 5 | 5 |
| 10 | 100 |

Beispiel 29

Erysiphe-Test (Gerste)/protektiv

Lösungsmittel : 100 Gewichtsteile Dimethylformamid
Emulgator : 0,25 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer rel. Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele :

Tabelle IV

Erysiphe-Test (Gerste)/protektiv

| Wirkstoff | Wirkstoffkonzentration in der Spritzbrühe in Gew.-% | Krankheitsbefall in % der unbehandelten Kontrolle |
|---|---|---|
| bekannt: | | |
| 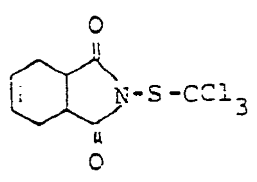 (Captan) | 0,025 | 100 |
| erfindungsgemäß: | | |
| 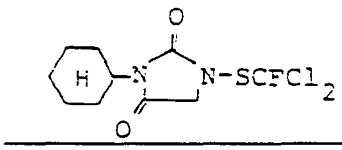 | 0,025 | 0,0 |

Beispiel 30

Fusarium nivale-Test (Roggen)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 × 100 Korn 1 cm tief in eine Standarderde und kultiviert ihn im Gewächshaus bei einer Temperatur von ca. 10 °C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigen bei diesem Test z. B. die Verbindungen gemäß folgender Herstellungsbeispiele :

Tabelle V

Fusarium nivale-Test (Roggen)/Saatgutbehandlung

| Wirkstoff | Wirkstoffaufwandmenge in mg/kg Saatgut | kranke Pflanzen in % der insgesamt aufgelaufenen Pflanzen |
|---|---|---|
| ungebeizt | - | 12,8 |
| bekannt: | | |
| 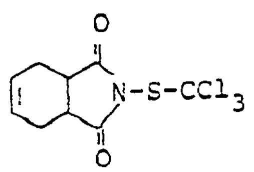 (Captan) | 500 | 5,4 |
| erfindungsgemäß: | | |
| 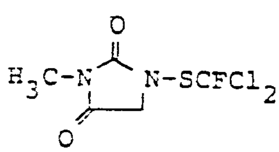 | 500 | 0,0 |

Beispiel 31

Pyricularia-Test (Reis)/protektiv

Lösungsmittel : 12,5 Gewichtsteile Aceton
Emulgator : 0,3 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß folgender Herstellungsbeispiele :

Tabelle VI

Pyricularia-Test (Reis)/protektiv

| Wirkstoffe | Wirkstoffkonzentration in % | Krankheitsbefall in % der unbehandelten Kontrolle |
|---|---|---|
| bekannt: | | |
| 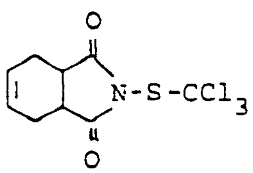 | 0,025 | 50 |
| erfindungsgemäß: | | |
| 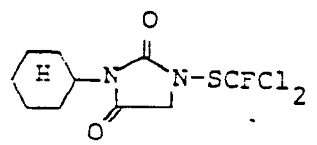 | 0,025 | 13 |

## Patentansprüche

1. N-sulfenylierte Hydantoine der Formel

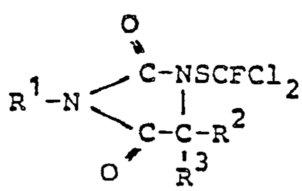 ,

in der

$R^1$ einen Alkylrest mit 1-12 C-Atomen, einen Alkenylrest mit 3-8 C-Atomen, einen Cycloalkylrest mit 5-12 C-Atomen oder einen Aralkylrest mit 6-10 C-Atomen im aromatischen Teil und 1-6 C-Atomen im aliphatischen Teil bedeutet, die gegebenenfalls durch 1-3 gleiche oder verschiedene substituenten aus der Gruppe Alkylreste mit 1-4 C-Atomen und/oder Fluor-, Chlor-, Brom- oder Iod-Atome substituiert sein können, wobei die Alkylsubstituenten noch durch Sauerstoff- oder Schwefelatome unterbrochen sein können, oder

$R^1$ für

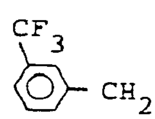

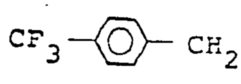

$C_6H_{11}CH_2$

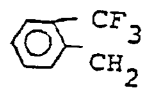

oder

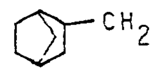

steht und

$R^2$ und $R^3$ gleich oder verschieden sind und für Wasserstoff, einen Alkylrest mit 1-12 C-Atomen oder einen Alkenylrest mit 3-8 C-Atomen stehen oder zu einem Cycloalkylrest mit 5-12 C-Atomen verbunden sind.

2. Verfahren zur Herstellung von N-sulfenylierten Hydantoinen des Anspruchs 1, dadurch gekennzeichnet, daß man Hydantoine der Formel

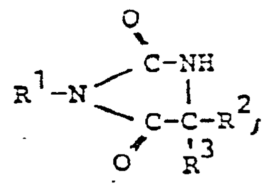

in der $R^1$, $R^2$ und $R^3$ die obengenannte Bedeutung haben, mit Dichlorfluormethansulfenylchlorid der Formel

$$FCl_2CSCl$$

in Gegenwart eines Verdünnungsmittels und eines säurebindenden Mittels umsetzt.

3. Verfahren nach Anspruch 2, dadurch gekennzeichnet, daß man die Umsetzung im Temperaturbereich von 0 bis 100 °C durchführt.

4. Verfahren nach den Ansprüchen 2 und 3, dadurch gekennzeichnet, daß man als säurebindende Mittel tert. Amine, Alkalihydroxide und/oder Alkalicarbonate einsetzt.

5. Verfahren nach den Ansprüchen 1 bis 4, dadurch gekennzeichnet, daß man 1 Mol des Hydantoins mit 1 bis 3 Mol des Dichlorfluormethansulfenylchlorids umsetzt.

6. Mikrobizide Mittel, enthaltend N-sulfenylierte Hydantoine des Anspruchs 1.

7. Verwendung von mikrobiziden Mitteln nach Anspruch 6 im Materialschutz.

8. Verwendung nach Anspruch 7, dadurch gekennzeichnet, daß die Anwendungskonzentrationen der N-sulfenylierten Hydantoine 0,001 bis 5 Gew.-%, bezogen auf das zu schützende Material, beträgt.

9. Verwendung der mikrobiziden Mittel nach Anspruch 6 im Pflanzenschutz.

**Claims**

1. N-Sulphenylated hydantoins of the formula

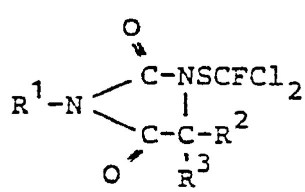

in which

$R^1$ denotes an alkyl radical with 1-12 C atoms, an alkenyl radical with 3-8 C atoms, a cycloalkyl radical with 5-12 C atoms or an aralkyl radical with 6-10 C atoms in the aromatic part and 1-6 C atoms in the aliphatic part, which may optionally be substituted by 1-3 identical or different substituents from the group comprising alkyl radicals with 1-4 C atoms and/or fluorine, chlorine, bromine or iodine atoms, it being possible for the alkyl substituents also to be interrupted by oxygen or sulphur atoms, or

$R^1$ represents

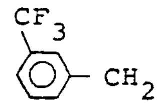

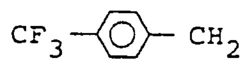

$C_6H_{11}CH_2$

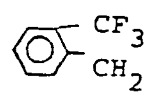

or

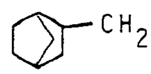

and $R^2$ and $R^3$ are identical or different and represent hydrogen, an alkyl radical with 1 to 12 C atoms or an alkenyl radical with 3-8 C atoms or are linked to form a cycloalkyl radical with 5-12 C atoms.

2. Process for the preparation of N-sulphenylated hydantoins of Claim 1, characterised in that hydantoins of the formula

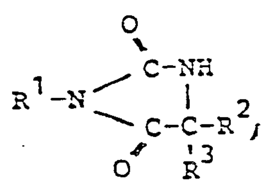

in which $R^1$, $R^2$ and $R^3$ have the abovementioned meaning, are reacted with dichlorofluoromethanesulphenyl chloride of the formula

$FCl_2CSCl$

in the presence of a diluent and an acid-binding agent.

3. Process according to Claim 2, characterised in that the reaction is carried out in the temperature range 0 to 100 °C.

4. Process according to Claims 2 and 3, characterised in that tert.-amines, alkali metal hydroxides and/or alkali metal carbonates are used as acid-binding agents.

5. Process according to Claims 1 to 4, characterised in that 1 mol of the hydantoin is reacted with 1 to 3 mols of the dichlorofluoromethanesulphenyl chloride.

6. Microbicidal agents, containing N-sulphenylated hydantoins of Claim 1.

7. Use of microbicidal agents according to Claim 6 in the protection of materials.

8. Use according to Claim 7, characterised in that the use concentrations of the N-sulphenylated hydantoins are 0.001 to 5 % by weight, based on the material to be protected.

9. Use of the microbicidal agents according to Claim 6 in plant protection.

**Revendications**

1. Hydantoïnes N-sulfénylées de formule :

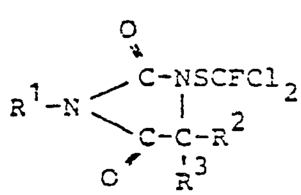

,

dans laquelle

$R^1$ signifie un radical alcoyle ayant 1 à 12 atomes de carbone, un radical alcényle ayant 3 à 8 atomes de carbone, un radical cycloalcoyle ayant 5 à 12 atomes de carbone ou un radical aralcoyle ayant 6 à 10 atomes de carbone dans la portion aromatique et 1 à 6 atomes de carbone dans la portion aliphatique, pouvant éventuellement être substitués par 1 à 3 substituants identiques ou différents du groupe des radicaux alcoyle ayant 1 à 4 atomes de carbone et/ou d'atomes de fluor, chlore, brome ou iode, les substituants alcoyle pouvant encore être interrompus par des atomes d'oxygène ou de soufre, ou bien $R^1$ représente

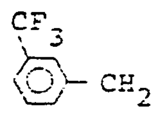

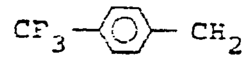

$C_6H_{11}CH_2$

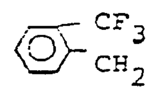

ou

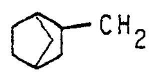

et $R^2$ et $R^3$ sont identiques ou différents et représentent de l'hydrogène, un radical alcoyle ayant 1 à 12 atomes de carbone ou un radical alcényle ayant 3 à 8 atomes de carbone, ou bien sont reliés en un radical cycloalcoyle ayant 5 à 12 atomes de carbone.

2. Procédé de préparation d'hydantoïnes N-sulfénylées selon la revendication 1, caractérisé en ce qu'on fait réagir des hydantoïnes de formule :

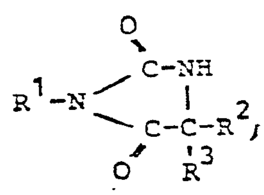

dans laquelle $R^1$, $R^2$ et $R^3$ ont la signification indiquée plus haut, avec du chlorure de dichlorofluorométhanesulfényle de formule :

$$FCl_2CSCl$$

en présence d'un diluant et d'un agent fixateur d'acide.

3. Procédé selon la revendication 2, caractérisé en ce qu'on exécute la réaction dans l'intervalle de température de 0 à 100 °C.

4. Procédé selon les revendications 2 et 3, caractérisé en ce qu'on utilise comme agents fixateurs d'acide des amines tertiaires, des hydroxydes alcalins et/ou des carbonates alcalins.

5. Procédé selon les revendications 1 à 4, caractérisé en ce qu'on fait réagir 1 mole de l'hydantoïne avec 1 à 3 moles du chlorure de dichlorofluorométhanesulfényle.

6. Agents microbicides contenant des hydantoïnes N-sulfénylées de la revendication 1.

7. Utilisation d'agents microbicides selon la revendication 6 dans la protection des matériaux.

8. Utilisation selon la revendication 7, caractérisée en ce que les concentrations d'application des hydantoïnes N-sulfénylées s'élèvent à 0,001 à 5 % en poids par rapport au matériau à protéger.

9. Utilisation des agents microbicides selon la revendication 6 dans la protection des plantes.